Europäisches Patentamt

European Patent Office

Office européen des brevets

(11) Veröffentlichungsnummer : **0 098 356**
**B1**

(12) EUROPÄISCHE PATENTSCHRIFT

(45) Veröffentlichungstag der Patentschrift :
22.10.86

(51) Int. Cl.⁴ : **C 07 D263/12//** C07D265/08

(21) Anmeldenummer : 83104262.7

(22) Anmeldetag : 30.04.83

(54) Verfahren zur Herstellung von reinen, wasserfreien Imidsäureestern durch Umsetzung von Nitrilen mit Aminoalkoholen.

(30) Priorität : 03.07.82 DE 3224880

(43) Veröffentlichungstag der Anmeldung :
18.01.84 Patentblatt 84/03

(45) Bekanntmachung des Hinweises auf die Patenterteilung : 22.10.86 Patentblatt 86/43

(84) Benannte Vertragsstaaten :
DE FR GB IT

(56) Entgegenhaltungen :
DE-A- 2 127 776
DE-A- 2 135 644
US-A- 3 741 961
JUSTUS LIEBIGS ANNALEN DER CHEMIE, 1974, Heft
6, Seiten 996-1009, Verlag Chemie, Weinheim, DE; H.
WITTE et al.: "Cyclische Imidsäureester aus Nitrilen
und Aminoalkoholen"
Die Akte enthält technische Angaben, die nach dem
Eingang der Anmeldung eingereicht wurden und die
nicht in dieser Patentschrift enthalten sind.

(73) Patentinhaber : HÜLS AKTIENGESELLSCHAFT
Patentabteilung / PB 15 - Postfach 13 20
D-4370 Marl 1 (DE)

(72) Erfinder : Kleine Homann, Walter, Dr.
Buchenallee 14
D-4408 Dülmen (DE)

**Beschreibung**

Cyclische Imidsäureester erhält man durch Umsetzung von Nitrilen mit Aminoalkoholen unter Abtreiben des gebildeten Ammoniaks bei Temperaturen zwischen 50 und 180 °C (DE-OS 21 27 776 = US-PS 3 813 378). Als Katalysatoren setzt man im Reaktionsmedium in ausreichendem Maße lösliche Metallsalze ein. Die Umsetzung erfolgt im allgemeinen bei Normaldruck. Man kann die Umsetzung auch bei erhöhtem oder vermindertem Druck durchführen. Nach der Lehre dieser Schrift kann das Arbeiten bei vermindertem Druck von Vorteil sein, weil dadurch das gebildete Ammoniak sofort aus dem Reaktionsmedium entfernt wird. Die sehr langen Reaktionszeiten sind jedoch ein Nachteil dieses Verfahrens.

Es besteht daher ein Interesse an einem verbesserten Verfahren, das bei erheblich kürzeren Reaktionszeiten höhere Ausbeuten bringt.

Die sich hieraus ergebende Aufgabe wird erfindungsgemäß entsprechend den Angaben der Patentansprüche gelöst.

Überraschenderweise wird das Ammoniak entgegen der Lehre der DE-OS 21 27 776 in einem bestimmten Druckbereich von 1,1 bis 10 bar, bevorzugt von 1,3 bis 6 bar, insbesondere von 1,5 bis 4 bar, sofort aus dem Reaktionsmedium entfernt. Es ist besonders überraschend, daß in diesem erfindungsgemäßen Druckbereich das Ammoniak schneller abgespalten wird und man bei wesentlich kürzeren Reaktionszeiten mindestens gleiche bzw. erheblich höhere Ausbeuten erreicht. Sogar mit stark reduzierten Katalysatorkonzentrationen erzielt man bei wesentlich kürzeren Reaktionszeiten erheblich höhere Ausbeuten.

Die Umsetzung erfolgt bei Temperaturen von 90 bis 230 °C, bevorzugt von 120 bis 200 °C.

Für das erfindungsgemäße Verfahren geeignete Nitrile sind Acetonitril, Propionitril, Butyronitril, iso-Butyronitril, Benzonitril und Adipodinitril.

Geeignete Aminoalkohole sind 2-Aminoethanol, 2-Aminopropanol-1, 1-Aminopropanol-2, 1-Aminopropanol-3 und 1-Aminobutanol-3.

Im allgemeinen setzt man 0,5 bis 1,0 Mol Aminoalkohol pro Mol Nitril bzw. halbem Mol Dinitril ein.

Als Katalysatoren sind im Reaktionsgemisch in ausreichendem Maße lösliche bzw. fein dispergierte Verbindungen des Lithiums, Kupfers, Calciums, Zinks, Cadmiums, Mangans, Eisen, Kobalt und/oder Nickel geeignet, beispielsweise die Oxide, Halogenide oder Carboxylate. Bevorzugt setzt man die Acetate ein. Die Menge der zugesetzten Katalysatoren beträgt vorzugsweise $10^{-5}$ bis $10^{-1}$ Mol/Mol Nitril.

Die Umsetzung erfolgt im allgemeinen ohne Lösungsmittel, man kann jedoch auch in einem inerten Lösungsmittel arbeiten. Das erfindungsgemäße Verfahren kann man kontinuierlich und auch diskontinuierlich durchführen.

Die Reaktionszeit beträgt 2 bis 18 Stunden. Damit werden die bisher erforderlichen Reaktionszeiten von 12 bis 41 Stunden stark verkürzt. Trotz der erheblich kürzeren Reaktionszeiten erreicht man beim erfindungsgemäßen Verfahren im allgemeinen wesentlich höhere Ausbeuten. Gegenüber dem Beispiel 14 der DE-OS 21 27 776 erzielt man beispielsweise bei einer wesentlich kürzeren Reaktionszeit, 5 Stunden statt 25 Stunden, eine relative Ausbeutesteigerung von 35 %, wobei die Katalysatorkonzentration nur ca. 2 % der dort beschriebenen beträgt.

Die Aufarbeitung der erhaltenen cyclischen Imidsäureester erfolgt in bekannter Weise, im allgemeinen durch eine fraktionierte Destillation, der sich eine Reinigung durch Umkristallisieren anschließen kann. Man erhält wasserfreie Imidsäureester mit einer sehr hohen Reinheit von 99 bis 99,9 %.

Die erfindungsgemäß hergestellten cyclischen Imidsäureester können zum Beispiel als Korrosionsschutzmittel, Antistatika oder selektive Lösungsmittel verwendet werden. Man kann sie auch gemäß DE-AS 12 06 585 (=GB-OS 1 086 572) zu den Poly[N-acyl]-ethyleniminen polymerisieren.

Beispiele

20 Mol des Nitrils werden mit 17,2 Mol des entsprechenden Aminoalkohols und 2 g (0,01 Mol) Zinkacetat mit $N_2$ auf den Reaktionsdruck gebracht und zum Rückfluß erhitzt. Die Destillation der Reaktionslösung führt zu reinen, wasserfreien (≥ 99,5 %) Oxazolinen.

(Siehe Tabelle Seite 3 f.)

**Patentansprüche**

1. Verfahren zur Herstellung von reinen, wasserfreien cyclischen Imidsäureestern durch Umsetzung von Nitrilen ausgewählt aus der Gruppe Acetonitril, Propionitril, Butyronitril, iso-Butyronitril, Benzonitril und Adiponitril mit Aminoalkoholen ausgewählt aus der Gruppe 2-Aminoethanol, 2-Aminopropanol-1, 1-Aminopropanol-2, 1-Aminopropanol-3 und 1-Aminobutanol-3 in Gegenwart von Metallverbindungen ausgewählt aus der Gruppe Verbindungen des Lithiums, Kupfers, Calciums, Zinks, Cadmiums, Mangans, Eisen, Kobald und/oder Nickel als Katalysator unter Abtreiben des gebildeten Ammoniaks, dadurch

| | Nitril | Amin | Reaktions- | | | Umsatz[2] | Ausbeute |
| | | | Temp. ($^O$C) | Druck (bar) | Zeit (h) | bezogen auf Nitril ($\%$) | ($\%$) |
|---|---|---|---|---|---|---|---|
| A | Propionitril[1] | 2-Aminoethanol | 110 | 1,0 | 24 | 48 | 56 |
| 1 | Propionitril | 2-Aminoethanol | 130 | 1,5 | 18 | 58 | 65 |
| 2 | Propionitril | 2-Aminoethanol | 140 | 1,9 | 11 | 62 | 78 |
| 3 | Propionitril | 2-Aminoethanol | 160 | 4,0 | 5 | 68 | 81 |
| 4 | Propionitril | 1-Aminopropanol-2 | 170 | 4,0 | 6 | 62 | 87 |
| 5 | Acetonitril | 1-Aminopropanol-2 | 165 | 4,0 | 6,5 | 53 | 75 |
| 6 | Acetonitril | 2-Aminoethanol | 150 | 4,0 | 5,5 | 69 | 74 |
| 7 | Butyronitril | 2-Aminoethanol | 190 | 4,0 | 7 | 67 | 67 |
| 8 | Benzonitril | 2-Aminoethanol | 190 | 4,0 | 7 | 80 | 80 |

[1] Vergleichsbeispiel
[2] Wegen des überschüssigen Nitrils beträgt der theoretische Umsatz 86 %.

0 098 356

gekennzeichnet, daß man die Umsetzung bei Temperaturen von 90 bis 230 °C und Drucken von 1,1 bis 10 bar durchführt.

2. Verfahren nach Anspruch 1, dadurch gekennzeichnet, daß man die Umsetzung bei einem Druck von 1,3 bis 6 bar, vorzugsweise bei 1,5 bis 4 bar, durchführt.

## Claims

1. A process for the production of a pure, waterfree, cyclic imido acid ester by reaction of a nitrile selected from acetonitrile, propionitrile, butyronitrile, isobutyronitrile, benzonitrile and adiponitrile with an aminoalcohol selected from 2-aminoethanol, 2-aminopropanol-1, 1-aminopropanol-2, 1-aminopropanol-3 and 1-aminobutanol-3 in the presence of a metal compound selected from compounds of lithium, copper, calcium, zinc, cadmium, manganese, iron cobalt and/or nickel as catalyst with separation of the ammonia generated, characterised in that the reaction is carried out at a temperature of 90 to 230 °C and a pressure of 1.1 to 10 bar.

2. A process according to claim 1, characterised in that the reaction is carried out at a pressure of 1.3 to 6 bar, preferably 1.5 to 4 bar.

## Revendications

1. Procédé de préparation à l'état pur d'imidoesters cycliques anhydres par réaction de nitriles choisis dans le groupe de l'acéto-nitrile, du propio-nitrile, du butyro-nitrile, de l'iso-butyro-nitrile, du benzo-nitrile et de l'adipo-nitrile sur des amino-alcools choisis dans le groupe du 2-amino-éthanol, du 2-amino-propanol-1, du 1-amino-propanol-2, du 1-amino-propanol 3 et du 1-amino-butanol 3, en présence de composés métalliques choisis dans le groupe des composés du lithium, du cuivre, du calcium, du zinc, du cadmium, du manganèse, du fer, du cobalt et/ou du nickel comme catalyseur, en chassant l'ammoniac formé, caractérisé par le fait que l'on effectue la réaction à des températures de 90 à 230 °C et sous des pressions de 1,1 à 10 bars.

2. Procédé selon la revendication 1, caractérisé par le fait que l'on effectue la réaction sous une pression de 1,3 à 6 bars, de préférence sous une pression de 1,5 à 4 bars.